# EUROPEAN PATENT APPLICATION

(11) **EP 1 925 200 A2**
(43) Date of publication of application: **28.05.2008**
(21) Application number: 07380322.3
(22) Date of filing: 21.11.2007
(51) Int. Cl.: A01M 1/02, A01M 1/20, A01M 29/00

(54) **Ceramic pheromone dispensers**

(30) Priority: 21.11.2006 ES 200602971
(71) Applicant: Rauschert España, S.L. Sociedad Unipersonal, 08185 Lliça del Vall (Barcelona) (ES)
(72) Inventor: Tico Maluquer, Lluis, 08185 Lliça del Vall (Barcelona) (ES); Olasz Quintana, Ernest, 08185 Lliça del Vall (Barcelona) (ES); Diaz Jaramillo, Pedro, 08185 Lliça del Vall (Barcelona) (ES)
(74) Representative: Carpintero Lopez, Francisco

(57) **Abstract**

The invention relates to a pheromone dispenser comprising a ceramic material part and a composition comprising said pheromone, as well as to a process for obtaining it. The invention also relates to the use of said dispenser in tracking and/or controlling said insect pests, such as Lobesia Botrana among others, in crops.

## Description

### FIELD OF THE INVENTION

The present invention relates to a ceramic pheromone dispenser, as well as a process for obtaining it. The invention also relates to a process for tracking and/or biologically controlling a pest comprising the use of said dispenser.

### BACKGROUND OF THE INVENTION

Chemical pesticides can be used to reduce or eliminate the harmful effects on certain crops caused by pests originated by insects, normally from the lepidopteran family, such as: Anarsia lineatella for almonds, Lobesia Botrana for vineyards, Chilo supresalis for rice, Cydia pomonella for apples and pears, and the dipteran Ceratitis capitata for citrus fruits and other fruit trees. The use of such pesticides, simultaneously to eliminating the insects that are harmful for the fruit, also causes collateral damage such as the contamination of the cultivated land, the effect on trophic chains, the contamination of aquifers or water currents. Pesticides therefore affect both fauna and soil, some effects being short-term and other effects being long-term.

In addition to pesticides, there are other methods for controlling pests. The use of the technique known as "sexual confusion" is thus known, consisting of releasing into the environment the pheromone emitted by the females of each insect species in such a way that the males have enormous difficulties in having sexual intercourse with the females of their species and therefore reducing the proliferation of the insect in question, but without completely eliminating it and without contaminating the environment with the harmful effects derived from pesticides. This system requires highly accurate mechanisms to maintain a suitable concentration in the environment, sufficient in amount and over time, to be effective while drastically reducing the insect population. The system can consist of a type of device which must be effective in the sense of dispensing the pheromone such that the confusion among insects is effective during the entire time that the insect is sexually active. Said system must advantageously not involve a large consumption of pheromones due to the important cost thereof, and must have necessary capacity so that this dispensing is maintained constant in the environment to reduce the possible mating and subsequent fertilization.

Some of the systems for dispensing pheromones currently existing in the state of the art are described in patents US 5,316,148, US 6,065,687 and US 4,160,335.

Currently used dispensers are generally complex systems that are mainly based on the use of sheets or products formed by different plastic layers or sheets with a controlled porosity or permeability, with or without grooves for achieving a regular dispensing of the active product.

There are several drawbacks inherent to said devices, including the natural thermal differences affecting the plastics of devices which must remain outside for a long time period. The duration of the active period is not constant and does not ensure the dispensing during the entire fertile period of the insect in question.

There is therefore still a need in the state of the art for alternative systems at least partly overcoming the drawbacks of the devices of the state of the art.

### BRIEF DESCRIPTION OF THE DRAWINGS

Figure 1 shows the evaporation curve of the FERO, CIBA and HMBP compositions in a controlled atmosphere using the G914 part. The x-axis shows the time (T) expressed in days and the y-axis shows the weight W expressed in grams.
Figure 2 shows the evaporation curve of the FERO, CIBA and HMBP compositions in a controlled atmosphere using the G928 part. The x-axis shows the time (T) expressed in days and the y-axis shows the weight W expressed in grams.

### DESCRIPTION OF THE INVENTION

In one aspect, the present invention provides a pheromone dispenser comprising a ceramic material part and a composition comprising a pheromone. Said dispenser is optionally provided with a securing means for placing it in the cultivated fields, particularly in the branches of shrubs, plants or trees which are to be treated.

The pheromone dispenser, hereinafter also dispenser of the invention, can be manufactured from a ceramic material with a controlled porosity which allows dispensing the pheromone in a regular, controlled and predetermined manner and for a sufficiently long time period to prevent the sexual activity of the insect or parasite to be controlled, in the fertility period of said species.

Obtaining the dispenser comprises the steps of:
(i) preparing a ceramic part;
(ii) impregnating said part with a composition comprising a pheromone; and
(iii) optionally incorporating a dispenser securing means for placing it in the field.

Step (i) is a conventional step for obtaining a ceramic part which can be carried out according to any method known in the state of the art. Step (i) particularly comprises in turn the following steps:
a) mixing the inorganic raw material and a pore-inducing material until the homogenization of the mixture:
b) adding to the previous mixture a mixture of water and other conventional additives in the obtaining of ceramic materials and mixing until obtaining a homogeneous mass again;
c) shaping a ceramic part from the mass obtained in the previous step;
d) drying the part obtained in the previous step;
e) sintering the dried part; and
f) finally machining until obtaining a ceramic part with the suitable dimensions.

The inorganic raw materials can be selected from clay with a high alumina content, talc, fired ground fireclay, calcium carbonate, sepiolite-type superplastic clay, Kieselguhr with a proportion of no more than 30% by weight of calcium carbonate and mixtures thereof. Said inorganic material can be added in different and variable proportions. They are introduced in a conventional mixer together with a pore-inducing material which can be selected, among other materials, from wheat, rice, almond shell, pine nut shell, walnut shell, olive stone, grape flours, fir flour and mixtures thereof. The pore-inducing material consists of organic material which is added to the raw inorganic material, having a particle size typically comprised between 100 and 400 microns to obtain a determined porosity at the end of the process. The mixture is mixed until its homogenization. The percentage of pore-inducing material with respect to the inorganic material can vary between wide margins. In a particular embodiment, almond shell, pine and rice flours are used in a proportion comprised between 20% and 40% by weight with respect to the weight of the ceramic mass.

In step b), a mixture of water and conventional additives in the field of ceramics, which are necessary and suitable for the subsequent shaping of the part according to the pre-established method, either dry pressing, extrusion or isostatic pressing, is added to the previous mixture. Said additives are selected from oils, such as Zusoplast 126/3 (preparation of fatty acids with non-ionic emulsions), Oleica (preparation of hydrocarbons and oleic acid), Smayh Oil R (mineral oil and saturated and unsaturated fatty acids, mainly oleic acid), Product KP5031, (preparation of hydrocarbons and paraffins), Dervacid 3290 (preparation of hydrocarbons and fatty acids) as well as chemical hardeners such as cellulose polymers, such as methylethylcellulose, hydroxyethylmethylcellulose and the like to achieve sufficiently high dry mechanical strength for its subsequent handling, lubricant such as charcoal from white woods, duly ground with a particle size of less than 50 microns, Formentrenol RST7NEU. The resulting mixture is mixed until its homogenization. The amount of added water is such that the final water content in the resulting homogenous mass is comprised between 15% and 35% by weight with respect to total weight of the homogeneous mass obtained.

The resulting homogenous mass is shaped in step c) according to any conventional method, such as by extrusion for example, to obtain a part with suitable dimensions and shape. In a preferred embodiment, the mass is extruded in the form of tubes. The shaped parts are dried by means of conventional methods in step d) until they have a residual water content of approximately 5% by weight with respect to the total weight of the part. They are then subjected to sintering at high temperatures and for a sufficient time period until reaching the suitable porosity; this step is carried out in special ovens with internal atmosphere, temperature and pressure controls of the oven, as well as the necessary controls for a controlled cooling at all its points for achieving a correct sintering of the material.

The sintered parts are finally machined until obtaining a ceramic part with the suitable dimensions and shape.

The characteristics of the ceramic parts for preparing pheromone dispensers, such as their chemical composition, their porosity and their average pore size, vary according to the nature of the starting material, the proportions thereof and the conditions of the process for obtaining them.

The dimensions and shape of the ceramic parts can vary within wide margins. In a particular embodiment, the ceramic parts are in the form of tubes with the following dimensions: an outer diameter between 7 and 10 mm; an inner diameter between 4 and 6.5 mm and a length between 20 and 35 mm. These dimensions are by no means limiting.

The porosity can also vary within wide margins. In a particular embodiment, it is comprised between 15% and 62%. The pore size can also vary between wide margins. In a particular embodiment, it is comprised between 1 µm and 10 µm, or it can have a dual mode distribution of 0.1 µm and 10 µm.

The ceramic part called G914 (obtained according to Example 1) as well as the part called G928 (obtained according to Example 2) and V990 have the characteristics of Table 1 below after sintering:

**Table 1**

| **Ceramic Part** | **Composition** | **Porosity Average** | **Pore Size** |
|---|---|---|---|
| G914 | Calcium aluminium oxide (CaAl₂O)₄ and Quartz | 49% | 1 µm |
| G928 | Mullite (Al₆Si₂O₁₃), quartz (SiO₂), Cristobalite and Tridymite (SiO₂) | 60 % | Dual-mode: 1µm and 10 µm |
| V 990 | Quartz (SiO₂), mullite (Al₆Si₂O₁₃), and Cristobalite and Tridymite(SiO₂) | 62 % | 0.1 µm |
| | | | |

In the previous table, the porosity in % relates to the percentage by volume of open pores.

Once the ceramic part has been obtained, it can be impregnated by immersing the part in a bath containing the composition comprising the pheromone for the necessary time period to achieve the absorption of the necessary amount of pheromone for the application for which it is intended and the duration of the release thereof over time.

Due to the high cost of pheromones, it is convenient to control, before the impregnation, that the porosity and average pore size are optimal for their specific application and that the dimensions of the dispensers do not have important variations among one another because the amount of composition comprising the pheromone impregnated in a part depends on these variables.

An amount of pheromone compositions can alternatively be deposited on the chosen ceramic part.

The amount of composition comprising pheromone which is impregnated in a ceramic part is physically limited by the filling of all the existing pores, i.e., it is limited by its volume multiplied by its porosity and by the density of the composition comprising pheromone to be impregnated. However, there is always a certain amount of pores which will never be filled with the composition due to their dimensions. It is calculated that, depending on the porosity and the average pore size, between 2% and 5% of open pores, which the pheromone cannot access due to their smaller size, can be discarded.

Generally, depending on the impregnation method and provided that this limit is not reached, the desired amount of composition can be impregnated, which amount can be easily calculated so that it is the necessary amount for the duration of the release of the pheromones in the field to be treated, without reaching the degradation or polymerization of the pheromones caused by the effects described below.

It must be taken into consideration that there is always a remaining amount, which is never dispensed, which moistens all the walls of the pores, i.e. the specific area of the ceramic material plus a small amount which is not percolated.

This amount is typically of no more than 5% by weight with respect to the total weight of impregnated composition, which will remain in the dispenser at the end of its use.

The type of pheromone used in the composition to be impregnated can initially be any pheromone according to the type of insect or parasite pest to be controlled.

In a particular embodiment, the insect to be controlled is the lepidopteran Lobesia Botrana or grape berry moth, which has currently acquired a pest character and is found in all Spanish viticultural regions except the island regions.

The pheromone used against this pest is (E, Z)-7,9-dodecadienyl acetate, which is obtained from synthesizing companies as a Pheromone for Lobesia Botrana, and with a 99.9% purity. Given that the half-life of pure pheromones and particularly of (E, Z)-7,9-dodecadienyl acetate is short (between 85 and 90 days), the ceramic parts are impregnated with compositions further comprising stabilizers protecting them from degradation by ultraviolet radiation and from oxidation by air.

In a particular embodiment, the ceramic part can be impregnated with any of the following prepared stabilized solutions: CIBA or HMBP or FERO, which does not comprise stabilizers. Their respective compositions are shown in Table 2 below:

**Table 2**

| Solution | Composition | Weight (%) |
|---|---|---|
| CIBA | (HMBT) [1] | 2.0 |
| | TBH [2] | 2.0 |
| | 99.9%Pheromone | 96.0 |
| HMBP | HMBP [3] | 2.0 |
| | TBH [2] | 2.0 |
| | 99.9%Pheromone | 96.0 |
| FERO | 99.9%Pheromone | 100.0 |

| | | |
|---|---|---|
| in which the pheromone is (E, Z)-7,9-dodecadienyl acetate; [1] HMBT is the compound 2-(2'-hydroxy-5'-methylphenyl) benzotriazole; [3] HMBP is the compound 2-hydroxy-4-methoxybenzophenone Both act as UV radiation protectors [2] TBH is the compound *tert*-butyl hydroquinone; and it has an antioxidizing function All of them are commercially available (Alfa Aesar or Ciba Especialidades Quimicas) | | |

The solutions of the previous table can be used to impregnate any type of ceramic part with any dimensions.

The release rate of the composition comprising pheromones from the dispensers and its dosing are determined by parameters such as the dimensions of the dispenser, the free contact surface of the dispenser with air, its porosity and average pore size characteristics, the type of composition comprising pheromone, as well as by the atmospheric conditions such as wind and temperature which cannot be regulated but only predicted for each case.

One of the advantages of the dispenser of the invention is that it is more stable than the dispensers of the state of the art in different atmospheric conditions due to the intrinsic heat-resistant and insulating character of the used ceramic material itself.

The dispenser of the invention can be easily designed by a person skilled in the art according to the pheromone to be used, the determined time period that the pheromone is to be released in a controlled manner, and can be designed taking into account the porosity and the suitable average pore size of the ceramic part for the specific application and the dimensions of the dispenser.

In a particular embodiment, the ceramic parts called G914 and G928 are impregnated with compositions comprising (E, Z)-7,9-dodecadienyl acetate: CIBA, HMBP and FERO, to give rise to the corresponding dispensers.

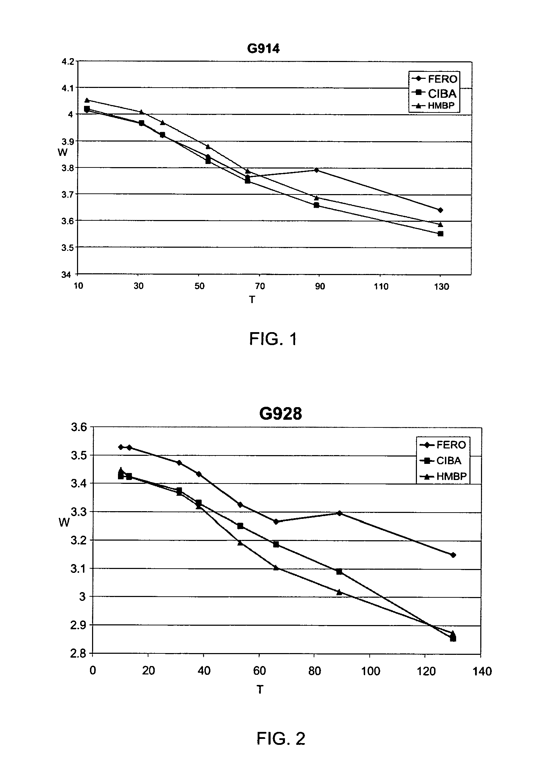

The release curves of said compositions in a controlled atmosphere against time are shown in Figures 1 and 2 respectively, in which a weight decrease in grams of the dispenser against time is shown. Under controlled atmosphere conditions refers to the fact that the impregnated dispenser is placed inside an oven under controlled temperature conditions, comprised between 22°C and 35°C, and an air renewal inside the oven of 3 times per second to evaluate the dispensing of the pheromone composition.

As can be verified, each dispenser releases the pheromone in a controlled manner; and the release rate varies among compositions (see Table 3). Said release rate also depends on the nature of the ceramic material (either G914 or G928). The rate will also depend on the dimensions of each dispenser. Said controlled release rate is shown in the following Table:

**Table 3**

| **Ceramic part** | **Solution** | **Slope** |
|---|---|---|
| G914 | CIBA | -4.2 mg/day |
| | HMBP | -4.3 mg/day |
| | FERO | -3.1 mg/day |
| G928 | CIBA | -4.8 mg/day |
| | HMBP | -5.0 mg/day |
| | FERO | -3.2 mg/day |

The dispenser of the present invention can be designed for the protection of the crop which is to be kept safe, such that it has the necessary volume, controllable porosity and dispenses the active product at the required rate to obtain the protection of the crop object of this treatment. Other additional advantages of the dispenser of the invention consist of the fact that, on one hand, its consistency is sufficient for its use and handling, that at the end of its work period it retains a minimum amount of active pheromone, and its ceramic material is inert and does interact physically or chemically with the composition comprising the pheromone. It is also stable to climate changes, such that its physical characteristics and its behavior due to moisture, climate, atmospheric changes, etc., remain invariable.

The ceramic components have a response against the solar heat that is very different from the plastic materials used in the state of the art, allowing a more uniform dispensing independent of the heat variations between sun-shade hours, between day and night and in the different seasons of the year in which the parasites to be controlled develop, normally spring and summer.

The heat-resistance and insulating property of the ceramic body make the heat accumulated during the day be released when the sun sets, at the time of the greatest sexual activity of the considered insects, at dusk, keeping the liquid at a more constant temperature, dispensing the pheromone more homogeneously.

An additional and determining advantage is that its composition is environmentally friendly, such that after the time of use the remains of the device do not pose any ecological or soil contamination problem. The use of ceramic materials as a base for the dispensers, instead of plastics and polymers from organic chemistry, allows progressing in the implementation of biological agriculture which is so respected by increasingly larger sectors of society.

In another additional aspect of the invention, the pheromone dispensers of the invention can be used for tracking and/or controlling pests in the fields, in plants, shrubs, trees such as vines, fruit trees, etc.

The dispenser of the invention is optionally provided with a securing means for placing it in the field. The securing means must advantageously be sufficiently fixed in the branches of the plants, shrubs or trees to be protected for example, taking into consideration the force of the wind in its strongest gusts in the treatment area. The securing means must preferably be simple and practical so it can be easily and quickly applied by the farmer for example, such that it advantageously does not require the use of both hands or an excessive amount of time. The securing means must preferably be resistant to degradation in the field as well as to oxidation, rain, high temperatures, such that detachment due to subsequent premature aging is prevented. The securing means is preferably formed by a little or non-contaminating material, and which is as cost-effective as possible.

In a particular embodiment, the securing means is elastic, such as for example a plastic element with sufficient elasticity so that upon forcing its introduction in the branch it opens, it is placed and recovers its initial position again.

In another particular embodiment, in order to use dispensers with two or more pheromones for effectively combating pests, two or more dispensers are placed in an element having exactly the same behavior as a "spring", so that once it is introduced in the branch it returns to the initial state, such as small bags containing several dispensers in their inside or in cavities, and so that this "spring" effect is formed between two of such bags, which "spring" is secured to the branch or support element, returning to its initial crossing and clasping position of the securing element.

In an additional aspect, the present invention provides a process for tracking and/or controlling pests in a field which comprises placing in the field one or more dispensers according to the present invention, optionally provided with a securing means, particularly in the branches of plants or trees to be treated, with the suitable pheromone for tracking or controlling the insect pest of interest.

Examples of the process of the invention are set forth below, which are described to better understand the invention and must by no means be considered as limiting the scope thereof.

### EXAMPLES

### Example 1: Process for obtaining a pheromone dispenser based on the ceramic part called G 914.

The following substances were mixed in a conventional dry double sigma mixer for a time period of less than 10 minutes:
- 60% by weight of mine Kieselguhr, with an impurity up to a maximum of 30% of calcium carbonate;
- 25% by weight of sepiolite; and
- 15% by weight of almond shell flour with a size between 100 and 250 microns as a pore-inducing agent.

Once the previous mixture has been homogenized in powder form, a mixture of water is prepared, to which 1% of Zusoplast 126/3 (preparation of fatty acids with non-ionic emulsions) and 1% of hydroxyethylmethylcellulose (all the percentages being expressed by weight with respect to the total weight of the mixture) have been added.

An amount of this mixture was added until obtaining a water content of 32% by weight of the total weight of the homogenous mass.

The resulting mixture was mixed for a time period of 25 minutes, and was poured into the mouth of a vacuum extruder (Dorso brand).

With a die for obtaining a tube with a diameter of more than 7.4 mm and a lower diameter of about 5.2 mm, parts were extruded in the form of tubes which were deposited in trays to dry them.

Once the parts were dry, which is equivalent to a residual moisture of less than 5% (by weight with respect to the total weight of the part), they were introduced in a chamber oven (Grüne brand) and were subjected for a period of about 6 hours to a temperature of 950 °C to obtain the desired porosity.

The tubes were then cut to a length of 35 mm and once they were at room temperature, they were impregnated with a composition comprising at least one pheromone. A securing element was previously introduced in each ceramic part (particularly for its securing to the branches of vines).

The tubes were then immersed in a solution called CIBA (see Table 2) for a time period of 4 hours, until achieving the necessary amount of pheromones by pore saturation, (440 mg per unit of ceramic part) and were allowed to drain off for 1 hour.

It was then placed in an oven under controlled temperature conditions, comprised between 22°C and 35°C and an air renewal inside the oven of 3 times per second to evaluate the dispensing of the pheromone composition.

The results obtained for the controlled release of composition are shown in Figure 1, showing the weight decrease (grams) of the ceramic dispenser against time (days).

### Example 2: Process for obtaining a pheromone dispenser based on the ceramic part called G 928

The following substances were mixed in a conventional dry double sigma mixer for a time period of less than 20 minutes:
- 20% by weight of fireclay;
- 40% by weight of calcined clay with a granulometry of less than 200 microns
- 40% by weight of almond shell flour with a size between 100 and 250 microns as a pore-inducing agent.

Once the previous mixture has been homogenized in powder form, a mixture of water is prepared, to which 5% of charcoal, 1 % of Zusoplast 126/3 (preparation of fatty acids with non-ionic emulsions) and 1 % of hydroxyethylmethylcellulose (all the percentages being expressed by weight with respect to the total weight of the mixture) have been added.

An amount of this mixture was added until obtaining a water content of 28% by weight of the total weight of the obtained mixture.

The resulting mixture was mixed for a time period of 15 minutes, and was poured into the mouth of a vacuum extruder (Dorso brand).

With a die for obtaining a tube with a diameter of more than 7.4 mm and a lower diameter of about 5.2 mm, parts were extruded in the form of tubes which were deposited in trays to dry them.

Once the parts were dry, which is equivalent to a residual moisture of less than 5% (by weight with respect to the total weight of the part), they were introduced in a chamber oven (Grüne brand) and were subjected for a period of about 6 hours to a temperature of 950 °C to obtain the desired porosity.

The tubes were then cut to a length of 35 mm and once they were at room temperature, they were impregnated with a composition comprising at least one pheromone. A securing element was previously introduced in each ceramic part (particularly for its securing to the branches of vines).

The tubes were then immersed in a composition comprising a pheromone and stabilizers, the solution called CIBA (see Table 2), for a time period of 4 hours, until achieving the necessary amount of pheromones by pore saturation, (440 mg per unit of ceramic part) and were allowed to drain off for 1 hour.

It was then placed in an oven in controlled temperature conditions, comprised between 22°C and 35°C and an air renewal inside the oven of 3 times per second to evaluate the dispensing of the pheromone composition.

The results obtained for the controlled release of composition are shown in Figure 2, showing the weight decrease (grams) of the ceramic dispenser against time (days).

Despite the fact that reference has been made to specific embodiments of the invention, it is evident for a person skilled in the art that the dispensers and the described process can be varied and modified in several ways, and that the mentioned details can be substituted by other technically equivalent details without departing from the scope of protection defined by the attached claims.

## Claims

1. A pheromone dispenser comprising a ceramic material part and a composition comprising a pheromone.

2. A dispenser according to claim 1, wherein the ceramic part is in the form of a tube with the following dimensions: an outer diameter between 7 and 10 mm; an inner diameter between 4 and 6.5 mm and a length between 20 and 35 mm.

3. A dispenser according to claim 1 or 2, wherein the porosity of the ceramic part is comprised between 15% and 62%.

4. A dispenser according to any of the previous claims, wherein the average pore size of the ceramic part is comprised between 1 µm and 10 µm, or has a dual mode distribution of 0.1 µm and 10 µm.

5. A dispenser according to claim 1, wherein the ceramic part has the following composition: calcium aluminium oxide, and quartz, a porosity of 49% and an average pore size of 1 µm.

6. A dispenser according to claim 1, wherein the ceramic part has the following composition: mullite, quartz, cristobalite and tridymite, a porosity of 60% and a dual mode average pore size distribution of 0.1 µm and 10 µm.

7. A dispenser according to claim 1 wherein the ceramic part has the following composition: mullite, quartz, cristobalite and tridymite, a porosity of 62% and an average pore size of 0.1 µm.

8. A dispenser according to any of the previous claims, wherein the pheromone is (E, Z)-7,9-dodecadienyl acetate.

9. A dispenser according to any of the previous claims, wherein the composition comprising a pheromone further comprises at least one stabilizing agent.

10. A dispenser according to claim 9, wherein the stabilizing agent protects the pheromone from ultraviolet radiation and/or from oxidation by air.

11. A dispenser according to claim 1, comprising a composition selected from the following compositions:
| Solution | Composition | Weight (%) |
|---|---|---|
| CIBA | (HMBT) [1] | 2.0 |
| | TBH [2] | 2.0 |
| | Pheromone | 96.0 |
| HMBP | HMBP [3] | 2.0 |
| | TBH [2] | 2.0 |
| | Pheromone | 96.0 |
| FERO | Pheromone | 100.0 |
and wherein the pheromone is (E,Z)-7,9-dodecadienyl acetate with a 99.9% purity.

12. A dispenser comprising a ceramic part having the following composition: calcium aluminium oxide, and quartz, a porosity of 49% and an average pore size of 1 µm and any composition according to claim 11.

13. A dispenser comprising a ceramic part having the following composition: mullite, quartz, and cristobalite, a porosity of 60% and a dual mode average pore size distribution of 0.1 µm and 10 µm and any composition according to claim 11.

14. A dispenser according to any of the previous claims, further comprising a securing means for placing it in a cultivated field.

15. A process for obtaining a dispenser according to any of the previous claims, comprising the steps of: (i) preparing a ceramic part; (ii) impregnating said ceramic part with a composition comprising a pheromone; and optionally (iii) incorporating a securing means for the dispenser.

16. A process according to claim 15, wherein step (i) comprises the steps of
a) mixing the inorganic raw material and a pore-inducing material until the homogenization of the mixture:
b) adding a mixture of water and other conventional additives to the previous mixture and mixing until obtaining a homogeneous mass again;
c) shaping a ceramic part from the mass obtained in the previous step;
d) drying the part obtained in the previous step;
e) sintering the part; and
f) finally machining the part until obtaining a ceramic part with the suitable dimensions.

17. A process according to claim 16, wherein the inorganic raw material is selected from clay with a high alumina content, talc, fired ground fireclay, calcium carbonate, sepiolite-type superplastic clay, Kieselguhr with a proportion of no more than 30% by weight of calcium carbonate and mixtures thereof.

18. A process according to claim 16, wherein the pore-inducing material is selected from wheat, rice, almond shell, pine nut shell, walnut shell, olive stone, grape, fir flours and mixtures thereof.

19. A process according to claim 18, wherein the pore-inducing material consists of almond shell, pine and rice flours in a proportion comprised between 20% and 40% by weight with respect to the weight of the ceramic mass.

20. A process according to claim 16, wherein the additives are selected from oils, chemical hardeners, lubricants and mixtures thereof.

21. A process according to claim 16, wherein the amount of added water is such that the final water content in the resulting homogenous mass is comprised between the 15% and 35% by weight with respect to the total weight of the homogeneous mass obtained.

22. A process according to claim 16, wherein step c) is carried out by extruding the homogenous mass in the form of tubes.

23. A process according to claim 16, wherein step d) of drying is carried out until achieving a residual water content of about 5% by weight with respect to the total weight of the part.

24. A process for obtaining a dispenser according to claim 15, wherein the step (ii) of impregnating the ceramic part with a composition comprising a pheromone is carried out by immersing the part in a bath containing said composition.

25. A process for obtaining a dispenser according to claim 15, wherein the step (ii) of impregnating the ceramic part with a composition comprising a pheromone is carried out by depositing the composition on the ceramic part.

26. The use of a dispenser according to any of claims 1 to 14, for tracking and/or controlling a pest in a cultivated field.

27. The use of a dispenser according to claim 26, for tracking and/or controlling an insect or parasite pest in plants, shrubs or trees.

28. The use according to the previous claim, wherein the insect is Lobesia Botrana and the cultivated field is a vineyard.

29. A process for tracking and/or controlling a pest in the field which comprises placing one or more dispensers according to any of claims 1 to14.
